# FASCICULE DE BREVET EUROPEEN

(11) **EP 1 802 270 B1**
(45) Date de publication et mention de la délivrance du brevet: **22.12.2010**
(21) Numéro de dépôt: 04816478.4
(22) Date de dépôt: 23.12.2004
(51) Int. Cl.: A61K 8/18, A61K 38/08, A61K 38/02

(54) **COMPOSITION DERMATOLOGIQUE ET/OU COSMETIQUE CONTENANT DES POLYPEPTIDES**
DERMATOLOGISCHE UND/ODER KOSMETISCHE ZUSAMMENSETZUNG MIT POLYPEPTIDEN
DERMATOLOGICAL AND/OR COSMETIC COMPOSITION CONTAINING POLYPEPTIDES

(30) Priorité: 18.02.2004 FR 0401593
(43) Date de publication de la demande: 04.07.2007
(73) Titulaire: Société d'Extraction des Principes Actifs SA (Vincience), 06904 Sophia Antipolis Cedex (FR)
(72) Inventeur: DAL FARRA, Claude, F-06650 Opio (FR); DOMLOGE, Nouha, F-06560 Valbonne (FR); BOTTO, Jean-Marie, F-06560 Valbonne (FR)
(74) Mandataire: Macquet, Christophe
(86) Numéro de dépôt international: PCT/FR2004/003357
(87) Numéro de publication internationale: WO 2005/089706

(56) Documents cités:
- WO-A-00/02577
- WO-A-00/06087
- WO-A-02/07754
- WO-A-2004/059001
- US-A1- 2003 036 646

## Description

La présente invention se situe dans le domaine de la pharmaceutique, et plus particulièrement dans le domaine de la dermatologie et de la cosmétologie.

La présente invention concerne l'utilisation de fragments polypeptidiques ou peptiques de protéines UCP, en tant qu'agent actif, seul ou en association avec au moins un autre agent actif, dans ou pour la préparation d'une composition pharmaceutique et/ou dermatologique et/ou cosmétique. L'invention est également relative à toute composition contenant ledit actif.

Le terme UCP désigne la famille des « UnCoupling Protein » ; ce sont des protéines découplantes : elles sont impliquées dans le découplage entre la réoxydation des coenzymes et la phosphorylation de l'ADP en ATP au niveau des mitochondries (Nicholls et al. Physiol. Rev., 64 : 1-64, 1984).

A ce jour, trois protéines de cette famille ont été identifiées : UCP-1, UCP-2 et UCP-3. L'UCP-1 a été la première protéine identifiée (Lin et al., FEBS Lett, 113 : 299-303, 1980). C'est une protéine découplante présente dans le tissu adipeux brun (Cassard et al. Journal of Cell biochemistry, 43, 1990). Cette forme de tissus adipeux est bien connue chez les petits mammifères, les animaux hibernants et les mammifères nouveau-nés ; c'est un organe thermogénique qui permet de résister au froid en produisant de la chaleur. La forte activité thermogénique des adipocytes bruns provient de la présence de UCP-1. Cette protéine dissipe une partie de l'énergie sous forme de chaleur par un découplage entre la respiration cellulaire et la synthèse d'ATP. En effet, l'UCP-1 est un transporteur de protons inséré dans la membrane interne des mitochondries. Lorsque la protéine est activée, elle catalyse la dissipation du gradient de protons à travers la membrane et court-circuite l'ATP-synthase. La respiration, n'étant plus couplée à la phosphorylation de l'ADP, devient un processus purement thermogénique. Le découplage de la respiration stimule l'oxydation des graisses et génère de la chaleur.

En plus de l'UCP-1, deux autres protéines découplantes, très proches de l'UCP-1, ont été identifiées : l'UCP-2 (Fleury et al. Nature Genetics, 15 : 269, 1997) que l'on trouve dans une large variété de tissus, dont l'intestin, les tissus adipeux, les muscles, le cerveau et les cellules du système immunitaire; et l'UCP-3 (Boss O et al., FEBS Lett, 1997) localisé essentiellement dans les muscles squelettiques et le tissu adipeux brun.

Ces protéines découplantes, spécialement UCP-2 et UCP-3, sont des protéines mal connues. Cependant des études démontrent qu'elles ont probablement un rôle important dans l'homéostasie énergétique (Klingenbeg, J. Bioenerg. Biomembr. 25: 447, 1993), notamment dans le métabolisme énergétique en général et dans le contrôle de l'efficacité métabolique alimentaire en particulier.

L'étude de ces protéines, et des gènes leur correspondant, impliqués dans le métabolisme énergétique, a fourni une nouvelle approche ainsi que de nouvelles cibles thérapeutiques permettant d'intervenir dans la régulation du métabolisme énergétique des mammifères, Des recherches ont notamment été menées dans le développement de médicaments qui agiraient, par thérapie génique, sur l'absence ou sur l'excès de protéines UCP. Les protéines UCP n'étant considérées jusqu'à ce jour sur un plan thérapeutique que comme des cibles potentielles au niveau génétique, afin de traiter des dysfonctionnements ou des maladies liées, entre autres, à l'obésité, au diabète ou encore à l'hyperlipidémie.

Ainsi, sur un plan thérapeutique, seule l'utilisation d'actifs capables d'agir au niveau de l'expression de ces protéines était envisagée. Ainsi, les brevets JP20031 13104 et JP2003 113106 décrivent, par exemple, des compositions à bases de plantes capables de stimuler l'expression de protéines UCP dans les adipocytes du tissu brun. Jamais les protéines UCP n'ont été utilisées, elles-même, en tant qu'agent actif.

Or, de manière inattendue et tout à fait surprenante, les inventeurs ont mis en évidence une activité thérapeutique et, plus particulièrement dermatologique et cosmétique, de ces fragments polypeptidiques ou peptidiques, notamment une activité lorsque ces fragments peptidiques sont appliqués sur la peau.

Les inventeurs ont notamment mis en évidence un effet amincissant localisé lorsque ces fragments peptidiques sont appliqués sur la peau. Plus particulièrement, il a été démonté que ces agents sont capables de réduire, d'éliminer ou de prévenir les surcharges graisseuses sous cutanées. Ils permettent ainsi d'ouvrir de nouvelles perspectives thérapeutiques et cosmétiques.

Ainsi, selon un premier aspect, la présente invention a pour objet l'utilisation de fragments peptidiques ou polypeptidiques de la famille des UCP on dérives biologiquement actif de ceux-ci, en tant qu'agent actif, seul ou en association avec au moins un autre agent actif, dans ou pour la préparation d'une composition pharmaceutique et/ou dermatologique et/ou cosmétique.

Tout particulièrement, l'invention est relative à l'utilisation de fragments peptidiques de la famille des UCP ou dérivés biologiquement actif de ceux-ci, en tant qu'agent actif amincissant, dans ou pour la préparation d'une composition dermatologique et/ou cosmétique.

Dans le cadre de l'invention, le fragment peptidique appartenant à la famille des UCP se caractérise notamment par le fait qu'il est choisi parmi les peptides correspondant à la séquence ID N°:
(1) Pro Leu Asp Thr Ala Lys Val Arg Leu Gln
(2) Pro Thr Glu Val Ala Lys Val Arg Phe Gln
(3) Pro Thr Asp Val Ala Lys Val Arg Leu Gln
(4) Pro Thr Glu Val Ala Lys Val Arg Leu Gln
(5) Pro Thr Asp Val Ala Lys Val Arg Phe Gln
(6) Pro Val Asp Val Val Lys Thr Arg Phe Ile
(7) Pro Val Asp Val Val Lys Thr Arg Tyr Met
(8) Pro Val Asp Val Val Lys Thr Arg Phe Met
(9) Pro Val Asp Val Val Lys Thr Arg Tyr Ile

Par ailleurs, selon une méthode de réalisation de l'invention toute particulièrement préférée, le fragment peptidique issu de la famille des UCP possède la séquence ID N°(1), c'est-à-dire la séquence Pro-Leu-Asp-Thr-Ala-Lys-Val-Arg-Leu-Gln.

Le terme « peptide » désigne un enchaînement de deux ou plusieurs acides aminés liés entre eux par des liaisons peptidiques ou par des liaisons peptidiques modifiées ; un polypeptide désignant un peptide de taille plus importante.

Par peptide, il faut entendre le peptide naturel ou synthétique de l'invention tel que décrit ci-dessus ou au moins l'un de ces fragments, qu'il soit obtenu par protéolyse ou de manière synthétique ou encore tout peptide naturel ou synthétique dont la séquence est totalement ou partiellement constituée par la séquence du peptide précédemment décrit.

Il se peut que pour des questions de résistance à la dégradation, il soit nécessaire d'utiliser une forme protégée du peptide selon l'invention. La forme de protection doit évidemment être une forme biologiquement compatible et doit être compatible avec une utilisation dans le domaine des cosmétiques ou de la pharmacie.

De nombreuses formes de protection biologiquement compatibles peuvent être envisagées, elles sont bien connues de l'homme du métier comme, par exemple, l'acylation ou l'acétylation de l'extrémité amino-terminale, ou l'amidation ou l'estérification de l'extrémité carboxy-terminale. Ainsi, l'invention concerne une utilisation telle que définie précédemment caractérisée par le fait que le peptide est sous forme protégée ou non. De préférence, on utilise une protection basée soit sur l'acylation ou l'acétylation de l'extrémité amino-terminale, soit sur l'amidation ou l'estérification de l'extrémité carboxy-terminale, soit encore des deux. Les dérivés d'acides aminés et les dérivés de peptides concernent aussi les acides aminés et les peptides reliés entre eux par une liaison pseudo-peptidique. On entend par "liaison pseudo-peptidique" tous les types de liaisons susceptibles de remplacer les liaisons peptidiques "classiques".

Dans le domaine des acides aminés, la géométrie des molécules est telle qu'elles peuvent théoriquement se présenter sous la forme d'isomères optiques différents. Il existe, en effet, une conformation moléculaire de l'acide aminé (AA) telle qu'elle dévie à droite le plan de polarisation de la lumière (conformation dextrogyre ou D-aa), et une conformation moléculaire de l'acide aminé (aa) telle qu'elle dévie à gauche le plan de polarisation de la lumière (conformation lévogyre ou L-aa). La nature n'a retenu pour les acides aminés naturels que la conformation lévogyre. En conséquence, un peptide d'origine naturelle ne sera constitué que d'acides aminés de type L-aa. Cependant la synthèse chimique en laboratoire permet de préparer des acides aminés ayant les deux conformations possibles. A partir de ce matériel de base, il est ainsi possible d'incorporer lors de la synthèse de peptide aussi bien des acides aminés sous forme d'isomères optiques dextrogyre ou lévogyre. Ainsi, les acides aminés constituant le peptide selon l'invention peuvent être sous configuration L- et D- ; de manière préférentielle, les acides aminés sont sous forme L. Le peptide selon l'invention peut donc être sous forme L-, D- ou DL-.

Les peptides selon l'invention peuvent être préparés par toutes méthodes appropriées, ainsi les peptides peuvent être des peptides isolés à partir de peptides et de protéines existant naturellement, des peptides recombinants, des peptides de synthèse, ou encore des peptides produits par une combinaison de ces méthodes. Bien entendu, les méthodes afin de préparer les peptides selon l'invention sont bien connues de l'homme du métier. Ainsi, le peptide selon l'invention peut être un peptide d'origine naturelle ou synthétique. Préférentiellement selon l'invention, le peptide est obtenu par synthèse chimique.

Selon un mode de réalisation avantageux de l'invention, les peptides précités, selon l'invention, sont préalablement solubilisés dans un ou plusieurs solvants cosmétiquement ou pharmaceutiquement acceptables, classiquement utilisés par l'homme du métier, comme l'eau, l'éthanol, le propylène glycol, le butylène glycol, le dipropylène glycol, les diglycols éthoxylés ou propoxylés, les polyols cycliques, la vaseline, une huile végétale ou tout mélange de ces solvants.

Selon encore un autre mode de réalisation avantageux de l'invention, les peptides précités sont préalablement solubilisés dans un vecteur cosmétique ou pharmaceutique comme les liposomes ou adsorbés sur des polymères organiques poudreux, des supports minéraux comme les talcs et bentonites, et plus généralement solubilisés dans, ou fixés sur, tout vecteur cosmétiquement ou pharmaceutiquement acceptable.

Il est bien entendu évident que le peptide selon l'invention peut être utilisé seul ou bien en association avec au moins un autre agent actif, dans ou pour la préparation d'une composition cosmétique et/ou dermatologique et/ou pharmaceutique.

Un aspect essentiel de l'invention est l'utilisation d'au moins un fragments de proteines de la famille des UCP ou dérivés biologiquement actif de ceux-ci, dans ou pour la préparation d'une composition cosmétique et/ou pharmaceutique, à usage topique, destinée, notamment, au traitement de la cellulite et/ou de la peau d'orange. Lesdits peptides étant aussi avantageusement utilisés afin de réduire, éliminer et/ou prévenir les surcharges graisseuses sous-cutanées.

Plus précisément, la présente invention vise l'utilisation d'au moins un fragment de proteines de la familla des UCP ou dérivés biologiquement actifs de ceux-ci, tels que définis précédemment, en tant qu'agent amincissant utilisable dans le domaine de la cosmétique notamment. L'invention concerne, de la même manière, l'utilisation des peptides, tels que définis précédemment, en tant qu'agent amincissant. Selon une méthode de réalisation actuellement préférée de l'invention, l'agent actif amincissant est le peptide possédant la séquence ID N°(1).

Ces peptides peuvent être ainsi utilisés dans ou pour la fabrication d'une composition cosmétique et/ou pharmaceutique, à usage topique, destinée au traitement de la cellulite et/ou au traitement de la peau d'orange. Ils sont utilisés d'une manière plus générale afin de réduire, éliminer ou prévenir les surcharges graisseuses sous-cutanées.

L'hypoderme est constitué de grosses cellules vacuolisées, les adipocytes, presque entièrement remplies de triglycérides. Ce tissu adipeux contient également du tissu conjonctif dans lequel se trouvent, entre autres, des fibroblastes particuliers et des préadipocytes. Ce tissu adipeux constitue le plus grand réservoir énergétique de l'organisme. Il est capable de stocker des lipides sous forme de triglycérides ou de les libérer sous forme d'acides gras et de glycérol. Les réserves lipidiques de l'organisme se renouvellent en permanence et il existe un équilibre, constamment adapté aux besoins énergétiques de l'organisme, entre le phénomène de lipolyse, qui libère des acides gras, et le phénomène de lipogénèse qui les stockent. Si un déséquilibre s'installe dans l'organisme entre ces deux phénomènes, les acides gras sont stockés dans les adipocytes, dont le volume et le nombre augmente : on parle d'hypertrophie et d'hyperplasie des adipocytes. Le développement excessif de la masse adipeuse peut alors se traduire par des modifications de l'aspect de la peau, voire évoluer vers une surcharge pondérale de l'individu, ou encore progresser vers une réelle obésité.

La cellulite est une configuration particulière du tissu adipeux, considérée de nos jours comme inesthétique. Elle désigne un aspect matelassé et capitonné de la peau qui correspond, de façon schématique, à l'accentuation de tissus adipeux dans certaines régions du corps, en particulier, chez la femme, au niveau des hanches, des cuisses, des fesses, des genoux et des avants-bras. A un stade avancé de la formation de cellulite, la peau prend spontanément l'aspect de « peau d'orange » ou capiton, qui se caractérise par une succession de petites bosses et dépressions dues à une traction de l'épiderme vers les tissus profonds.

Il a été constaté que les peptides selon l'invention, ou la composition les contenant, possèdent une action très efficace au niveau des adipocytes. En effet, ils contribuent à faire diminuer significativement la quantité de triglycérides contenue dans les adipocytes de l'hypoderme.

Ce phénomène est dû vraisemblablement à un blocage du phénomène de la lipogénèse, c'est-à-dire au blocage du processus de stockage des triglycérides qui aboutit à une hypertrophie des adipocytes. Une maîtrise de la lipogénèse, c'est-à-dire de la réaction de synthèse des triglycérides dans les adipocytes, permettra d'éviter une hypertrophie des adipocytes ainsi qu'une hyperplasie consécutive. Ainsi, lorsque, en cours de différenciation adipocytaire, la quantité de triglycérides présente dans les vacuoles n'augmente pas, le volume des adipocytes et leur nombre n'augmentent pas non plus. La peau reprend ainsi, petit à petit, son aspect « normal » : le tissu cellulitique ne se développe plus, l'effet peau d'orange est atténué. L'aspect disgracieux du corps s'estompe peu à peu.

L'actif selon l'invention permet ainsi de prévenir l'apparition de la cellulite ainsi que de lutter contre son aggravation.

Selon un autre aspect, l'invention concerne une composition cosmétique et/ou dermatologique et/ou pharmaceutique **caractérisée en ce qu**'elle contient, dans un milieu acceptable, comme principe actif, des fragments de protéines de la famille des UCP, ou peptides tels que définis précédemment.

D'une manière avantageuse la composition selon l'invention contient un ou plusieurs peptides choisis parmi les peptides correspondant aux séquences ID N° (1) à ID N° (9). Selon une réalisation particulièrement avantageuse de l'invention, la composition contient le peptide de séquence Pro-Leu-Asp-Thr-Ala-Lys-Val-Arg-Leu-Gln. D'une façon plus générale, la composition selon l'invention contient le composé actif tel que défini précédemment.

Dans la composition selon l'invention, le peptide peut être un mélange de dérivés peptidiques et/ou constitué de dérivés d'acides aminés. Il est bien entendu que le peptide selon l'invention peut être utilisé seul ou en association avec au moins un autre agent actif.

En particulier, l'invention concerne une composition cosmétique destinée à obtenir une action amincissante, et/ou destinée à réduire, éliminer ou à prévenir les surcharges graisseuses sous-cutanées, **caractérisée en ce qu**'elle comprend une quantité cosmétiquement efficace d'au moins un des fragments protéiniques tels que définis précédemment, afin d'obtenir ladite action amincissante et/ou afin de réduire, éliminer ou prévenir les surcharges graisseuses sous-cutanées. La composition selon l'invention est aussi avantageusement destinée à lutter contre la cellulite et/ou destinée à faire diminuer ou disparaître le phénomène de peau d'orange.

La composition contenant le peptide selon l'invention peut être une composition cosmétique ou dermatologique ou pharmaceutique. Préférentiellement selon l'invention, la composition est une composition cosmétique, car elle est destinée à améliorer l'aspect et les performances cutanées générales de l'individu qui en fait usage. La composition selon l'invention est préférentiellement une composition cosmétique et/ou dermatologique adaptée à l'administration par voie topique cutanée comprenant un milieu cosmétiquement ou pharmaceutiquement acceptable.

Il est bien évident que l'invention s'adresse aux mammifères en général et plus particulièrement aux êtres humains.

A titre d'exemple, la composition selon l'invention peut être appliquée localement sur les zones du visage ou du corps à affiner, en particulier sur les hanches, les fesses, les cuisses, le ventre ou le visage. Un des nombreux avantages de la présente invention est de pouvoir disposer d'un traitement par voie topique efficace contre l'adiposité tout en employant des méthodes « douces ».

La quantité efficace de principe actif correspond à la quantité nécessaire afin d'obtenir le résultat désiré.

Selon un mode de réalisation avantageux de l'invention, le peptide précité est présent dans les compositions de l'invention à une concentration comprise entre 0,005 et 500 ppm (parties par million) environ, et préférentiellement à une concentration comprise entre 0,1 et 50 ppm environ par rapport au poids total de la composition finale.

Quelle que soit la forme de l'invention, la composition selon l'invention peut être injectée ou appliquée sur la peau (sur toute zone cutanée du corps), les cheveux, les ongles ou les muqueuses. Selon le mode d'administration, la composition selon l'invention peut se présenter sous toutes les formes galéniques normalement utilisées.

Préférentiellement, les compositions selon la présente invention se présenteront sous une forme galénique adaptée à l'administration par voie topique cutanée, et couvrent toutes les formes cosmétiques ou dermatologiques. Ces compositions doivent donc contenir un milieu cosmétiquement acceptable, c'est-à-dire compatible avec la peau , les muqueuses et les phanères.

Ces compositions pourront notamment se présenter sous forme d'une solution aqueuse, hydroalcoolique ou huileuse ; d'une émulsion huile-dans-eau, eau-dans-huile ou émulsions multiples ; elles aussi peuvent se présenter sous forme de crèmes, de suspensions, ou encore de poudres, adaptées à une application sur la peau, les muqueuses, les lèvres et/ou les phanères. Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick ou être appliquées sur la peau sous forme d'aérosol. Elles peuvent être utilisées comme produit de soin et/ou comme produit de maquillage de la peau.

Ces compositions comprennent, en outre, tout additif communément utilisé dans le domaine d'application envisagé ainsi que les adjuvants nécessaires à leur formulation, tels que des solvants, des épaississants, des diluants, des anti-oxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc.

Dans tous les cas, l'homme du métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre de 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Bien entendu, l'homme du métier veillera à choisir les éventuels composés complémentaires, actifs ou non-actifs, et/ou leurs quantités, de telle sorte que les propriétés avantageuses du mélange ne soient pas, ou sensiblement pas, altérées par l'adjonction envisagée.

Selon l'invention, on peut ajouter à la composition de l'invention d'autres agents actifs destinés, entre autres, au traitement de la cellulite et au phénomène de « peau d'orange ». Préférentiellement, la composition à visée amincissante selon l'invention contiendra, par exemple, avantageusement, en plus de l'actif tel que défini précédemment, un actif favorisant la lipolyse.

Les compositions selon l'invention trouvent une application notamment comme compositions cosmétiques ou pharmaceutiques pour la peau, les muqueuses et/ou les semi-muqueuses. Elles trouvent une application en tant que produit de protection et/ou de soin de la peau, ou encore en tant que composition anti-ride et/ou anti-âge, mais surtout elles trouvent une application toute particulière en tant que composition amincissante et/ou raffermissante. La composition amincissante et/ou raffermissante pourra être appliquée localement sur les zones du visage ou du corps à affiner, en particulier sur les hanches, les fesses, les cuisses, le ventre ou le visage. On peut également envisager d'autres applications dans le domaine des compositions en association, par exemple, avec d'autres agents actifs.

La présente invention concerne aussi un procédé de soin cosmétique afin d'obtenir une action amincissante, ainsi qu'un procédé de soin cosmétique destiné à réduire, éliminer et/ou prévenir les surcharges graisseuses sous-cutanées. Ce procédé est par ailleurs destiné à lutter contre la cellulite et/ou à lutter contre le phénomène de peau d'orange. Ce procédé consiste à appliquer, topiquement, sur les zones concernées de la peau du visage et/ou du corps, une quantité efficace des peptides, selon l'invention, tels que définis précédemment ou d'une composition, telle que définie précédemment, les contenant.

Le procédé de traitement cosmétique de l'invention peut être mis en oeuvre notamment en appliquant les compositions cosmétiques telles que définies ci-dessus, selon la technique d'utilisation habituelle de ces compositions, par exemple : application de crèmes, de gels, de sérums, de lotions, de laits, de shampooings ou de compositions anti-solaires sur la peau. Des modes de réalisation particuliers de ce procédé de traitement cosmétique résultent également de la description précédente.

D'autres avantages et caractéristiques de l'invention apparaîtront mieux à la lecture des exemples donnés à titre illustratif et non limitatif

### Exemple 1 : Mise en évidence de l'activité des peptides selon l'invention sur les adipocytes.

### I. Culture cellulaire :

Des cellules de la lignée cellulaire préadipocytaire 3T3-L1, non différenciées, sont ensemencées dans des LabTek 8 puits (pour la coloration Oil Red), dans des plaques 12 puits (pour le dosage de l'ATP), dans des plaques 24 puits (pour le dosage de l'AMPc) et dans des plaques 6 puits (pour le dosage du glycérol) ainsi qu'avec du milieu de culture DMEM (4,5 g/l) contenant des antibiotiques. Ces préadipocytes 3T3-L1 étant capables de rentrer, dans certaines conditions, en phase de différenciation terminale.

### II. Différenciation des adipocytes :

Une fois que les cellules 3T3-L1 ont atteint une confluence de 100 %, ces cellules sont différenciées par une culture dans différents milieux, sur une période allant de 6 à 8 jours.

Durant les 2 premiers jours, les cellules sont cultivées en présence d'IBMX, de dexaméthazone et d'insuline, dilués dans du milieu de culture DMEM 4,5 g/l. Puis elles sont cultivées durant deux jours en présence, uniquement, d'insuline diluée dans du milieu de culture DMEM 4,5 g/l et, enfin, les cellules sont cultivées durant 2 à 3 jours en milieu de culture contenant exclusivement du DMEM 4,5 g/l.

La différenciation des cellules en adipocytes matures est visualisée par l'apparition de gouttelettes lipidiques dans le cytoplasme des cellules.

L'effet du peptide ainsi que son impact sur les cellules 3T3-L1 différenciées ou en cours de différenciation a été évalué au travers de différentes techniques :
- la coloration « Oil Red »,
- le dosage de la quantité de l'ATP intracellulaire,
- le dosage de la quantité de l'AMP cyclique intracellulaire,
- le dosage du glycérol relargué par les cellules.

### III. La Coloration "Oil Red":

Le principe de ce test d'évaluation de l'effet des peptides repose sur une observation microscopique du nombre et de la taille des vacuoles lipidiques des adipocytes après coloration. Les adipocytes étant incubés ou non, en présence de la substance à tester.

Les cellules 3T3-L1, ensemencées dans des Lab-teks 8 puits, sont traitées avec le peptide de séquence ID N°(1), c'est-à-dire de séquence Pro-Leu-Asp-Thr-Ala-Lys-Val-Arg-Leu-Gln, représentatif de la famille de peptide selon l'invention, mis en solution à 0,5 % d'une solution à 50 ppm. L'actif a été ajouté à différents stades de culture :
- Dès l'ensemencement et jusqu'à la fin de la différenciation adipocytaire (72 heures de culture jusqu'à 100 % de confluence puis pendant les 6 jours de différenciation) ;
- 6 heures après l'ensemencement et jusqu'à la fin de la différenciation des cellules (66 heures de culture jusqu'à 100 % de confluence puis pendant les 6 jours de différenciation) ;
- Une fois les cellules différenciées en adipocytes, les cellules étant traitées avec l'actif durant 6, 24, ou 48 heures.

Après ces différentes périodes d'incubation, en présence ou non de l'actif à tester, les 3T3-L1 sont colorées par la technique « Oil Red ». La solution « Oil Red » (*Sigma,* O-0625) est préparée par ajout de 0,5 g de produit dans 100 mL d'isopropanol et par dilution au 4/10 dans de l'eau distillée, puis filtration. Les cellules sont fixées durant 10 minutes dans une solution de formol 4 % et NaCl, la solution Red Oil est appliquée durant 15 minutes. Une contre-coloration à l'Hématoxyline durant 30 secondes est possible. Les cellules sont ensuite rincées avec de l'eau tiède et montées sur lames, en milieu hydrophile (*Aquatex*). L'observation est effectuée au microscope optique de façon à distinguer les vacuoles graisseuses colorées en rouge.

Les résultats de l'observation des cellules démontrent que les cellules qui ont été traitées avec le peptide bien avant le début de la différenciation (c'est-à-dire dès leur ensemencement, ou bien 6 heures après leur ensemencement) ont une morphologie moins arrondie et un contenu en vésicules lipidiques intra-adipocytaires nettement diminué par comparaison avec les cellules témoins (sur lesquelles l'actif n'a pas été appliqué). Cette même observation est aussi effectuée par comparaison avec les adipocytes matures, c'est-à-dire différenciés, sur lesquels l'actif a été appliqué, qui ont une forme volumineuse sphérique et une accumulation importante de vésicules lipidiques intra-cytoplasmiques.

La solution contenant le peptide selon l'invention s'avère donc particulièrement efficace au niveau des adipocytes en cours de différenciation et non pas au niveau des adipocytes matures qui ont déjà accumulé des triglycérides dans leurs vésicules intra-adipocytaires. En effet, aucune diminution n'a été observée lorsque l'actif a été appliqué une fois la différenciation terminée.

### IV. Dosage de l'AMP cyclique intracellulaire :

L'objectif de ce test est de mesurer la variation de la concentration d'AMPc intracellulaire dans les adipocytes, afm d'en déduire une éventuelle activation du phénomène de lipolyse.

Le test a été effectué sur les cellules 3T3-L1 différenciées. Ces adipocytes sont traités avec le peptide de séquence ID N°(1), c'est-à-dire de séquence Pro-Leu-Asp-Thr-Ala-Lys-Val-Arg-Leu-Gln, représentatif de la famille de peptide selon l'invention, mis en solution à 0,5 % d'une solution à 50 ppm, pendant des périodes de 15 minutes, 30 minutes ou 1 heure. En parallèle des cellules 3T3-L1 différenciées sont également traitées avec de l'isoprotérénol (un agent inducteur de la lipolyse), à 1 µM, constituant ainsi un contrôle positif.

Après les différentes périodes d'incubation, en présence de l'actif à tester ou en présence d'isoprotérénol, la quantité d'AMPc contenue dans les adipocytes est mesurée par le biais du Kit « cAMP Biotrak® EIA System » de Amersham Biosciences ainsi que sur un principe colorimétrique de type ELISA. Le kit permet la lyse des cellules par une solution permettant ainsi l'hydrolyse des membranes cellulaires et le relargage de l'AMPc intracellulaire dans le milieu. Puis la fixation sur l'anticorps de capture de l'AMPc intracellulaire, extrait des échantillons, est mise en compétition avec de l'AMPc couplé à une enzyme, la peroxydase. Ainsi, après une révélation avec le substrat TMB, plus il y aura d'AMPc dans les échantillons à doser, plus le signal sera faible.

Les résultats obtenus démontrent qu'en présence de l'actif, il n'y a aucune augmentation de la quantité d'AMPc intracellulaire sur des cellules différenciées, par rapport à la condition contrôle, c'est-à-dire par rapport aux cellules traitées avec l'isoprotérénol.

### V. Dosage de fats intracellulaire :

Le but de cette étude est de déterminer l'influence du peptide selon l'invention sur la quantité d'ATP intracellulaire.

Cette étude s'effectue à l'aide d'un Kit "ATP Bioluminescence Assay Kit HS II". Les cellules 3T3-L1 différenciées sont traitées avec une solution à 0,5 %, d'une solution à 50 ppm, contenant le peptide de séquence ID N°(1), représentatif de la famille de peptide selon l'invention, pendant une période allant jusqu'à 96 heures. A la fin du temps d'incubation, les puits sont vidés de leur milieu et rincés avec 2 ml de PBS froid avant d'ajouter 250 µl d'un tampon de lyse fournis par le kit. Les cellules sont ensuite scrapées puis récoltées, séparément, dans des tubes 14 ml. Chaque puits est rincé avec 2 x 500 µl PBS froid et le tout est de nouveau récolté dans les tubes respectifs. Chaque tube est ensuite passé au polytron durant 10 secondes à 18000 tours/minute. A partir de ces échantillons, une dilution est réalisée au 1/12000^{ième} dans du PBS froid avant chaque lecture. Le dosage d'ATP est réalisé sur ces échantillons : 50 µL de cette dilution sont déposés dans une 1uma-cuvette et 50 µL de luminol sont ajoutés. Après 10 secondes, la lecture de la luminescence est déclenchée. Les valeurs sont standardisées par rapport à la quantité de protéines pour chaque échantillon. Les mesures sont effectuées à l'aide d'un appareil : le Biocounter M2010A LUMAC®/3M.

Les résultats obtenus démontrent qu'il n'y a aucune augmentation de la quantité d'ATP intracellulaire. Les mesures obtenues mettent même en évidence une diminution de la quantité d'ATP intracellulaire au bout de 96 heures de culture, sur des cellules différenciées et traitées par l'actif, en comparaison avec le contrôle.

### VI. Dosage du glycérol :

Le but de cette étude est de déterminer l'influence du peptide selon l'invention sur le relargage du glycérol par les adipocytes. Ce glycérol étant le résultat de la lipolyse.

Les cellules 3T3-L1 différenciées sont traitées à la dose de 0,5 % d'une solution à 50 ppm contenant le peptide de séquence ID N°(1), représentatif de la famille de peptide selon l'invention, durant des périodes de 1h30, 3h, 5h, 7h et 24 heures.

Le dosage du glycérol est réalisé à l'aide d'une réaction enzymatique en cascade aboutissant à la formation de NADH dont la quantité produite est évaluée par lecture spectrophométrique à 340 nm, la formation de NADH étant proportionnelle à la quantité de glycérol relargué dans le milieu de culture. Un contrôle interne de glycérol à 1mM a aussi été utilisé. Les valeurs sont standardisées par rapport à 1a quantité de protéines pour chaque échantillon.

Les résultats obtenus nous permettent de constater qu'aux différents temps étudiés, le relargage du glycérol est identique pour les puits contrôles et les puits traités avec l'actif à 0,5 % sur des cellules différenciées.

### VII. Conclusions :

Les résultats du test de la coloration "Oil Red" nous permet de conclure que l'actif selon l'invention a un effet particulièrement efficace au niveau de la diminution de la formation de gouttelettes lipidiques intra-adipocytaires. Cependant, cet effet n'est seulement observé que lorsque l'actif est appliqué avant le début et/ou pendant la différenciation cellulaire, aucune diminution n'étant observée lorsque l'actif est appliqué sur des adipocytes matures. Ces résultats suggèrent ainsi que le peptide agit au niveau de la lipogénèse et non au niveau de la lipolyse.

Les augmentations des quantités d'AMPc et d'ATP intracellulaire sont des indicateurs de l'activation de la voie de la lipolyse, le taux d'AMPc intracellulaire régulant l'activité de la Triglycéride-lipase, l'enzyme permettant l'hydrolyse des triglycérides. Ainsi, une non-augmentation des quantités d'AMPc et d'ATP intracellulaire suggère que l'actif selon l'invention n'agit pas en augmentant le phénomène de lipolyse. Ces résultats sont, par ailleurs, confirmés par la non-augmentation du relargage du glycérol dans 1e milieu extracellulaire, le glycérol étant un produit de la lipolyse.

Ces résultats tendent ainsi à démontrer que les peptides selon l'invention possèdent une action réellement efficace au niveau des adipocytes, et qu'ils s'avèrent tout particulièrement efficaces dans le processus de limitation de l'hypertrophie adipocytaire.

L'actif selon l'invention empêche ainsi l'accumulation de gouttelettes lipidiques dans les adipocytes, et permet ainsi, plus ou moins directement, une diminution de la masse adipeuse et une inhibition de son développement.

Cette diminution des vacuoles lipidiques dans les adipocytes non matures, combinée aux résultats des dosages d'AMPc, d'ATP et de glycérol, permet de conclure que les peptides selon l'invention agissent, au niveau des adipocytes, sur le mécanisme de la lipogénèse et non pas sur le mécanisme de lipolyse. Les peptides selon l'invention favorisent donc la non-accumulation des triglycérides contenus dans les vésicules intra-adipocytaires.

### Exemple 2 - Préparation de compositions.

### 1. Crème amincissante

| ***Noms commerciaux*** | ***Noms INCI*** | ***% massique*** |
|---|---|---|
| ***PHASE A*** | | |
| Montanov 68 | Cetearyl Alcohol (and) Cetearyl Glucoside | 5.00 |
| Squalane | Squalane | 2.50 |
| DUB IPP | Isopropyl Palmitate | 3.50 |
| Eutanol G | Octyldodecanol | 1.50 |
| Phenonip | Phenoxyethanol (and) Methylparaben (and) Ethylparaben (and) Butylparaben (and) Propylparaben (and) Isobutylparaben | 0.50 |

| ***PHASE B*** | | |
|---|---|---|
| Eau déminéralisée | Aqua (Water) | Qsp |
| Glycerine | Glycerin | 3.00 |
| Butylene Glycol | Butylene glycol | 3.00 |

| ***PHASE C*** | | |
|---|---|---|
| Simulgel EG | Sodium Acrylate/Acryloyldimethyl Taurate Copolymer(and) Isohexadecane(and) Polysorbate 80 | 0.60 |

| ***PHASE D*** | | |
|---|---|---|
| Peptide ID N°(1) | | 1.25 ppm |
| Parfum | Parfum (Fragrance) | Qsp |
| Colorant | | Qsp |

Les constituants de la phase A sont fondus à 75°C et les constituants de la phase B chauffés à 75°C. La phase A est émulsionnée à B, puis le mélange est refroidi en dessous de 40°C. Les phases C et D sont ensuite additionnées sous agitation constante.

### 2. Spray Raffermissant - Amincissant

| ***Noms commerciaux*** | ***Noms INCI*** | ***% massique*** |
|---|---|---|
| ***PHASE A*** | | |
| Emulgade SEV | Glyceryl Stearate (and) Ceteareth-20 (and) Ceteareth-12 (and) Cetearyl Alcohol | 4.60 |
| Eumulgin B2 | Ceteareth-20 | 1.40 |
| Cetiol OE | Dicaprylyl Ether | 3.00 |
| DUB B1215 | C12-C15 Alkyl Benzoate | 5.00 |
| Phenonip | Phenoxyethanol (and) Methylparaben (and) Ethylparaben (and) Butylparaben (and) Propylparaben (and) Isobutylparaben | 0.50 |
| DUB ININ | Isononyl Isononanoate | 5.00 |
| Phenonip | Phenoxyethanol (and) Methylparaben (and) Ethylparaben (and) Butylparaben (and) Propylparaben (and) Isobutylparaben | 0.50 |

| ***PHASE B*** | | |
|---|---|---|
| Eau déminéralisée | Aqua (Water) | 15.00 |
| Glycerine | Glycerin | 3.00 |
| ***PHASE C*** | | |
| Eau déminéralisée | Aqua (Water) | Qsp |

| ***PHASE D*** | | |
|---|---|---|
| Peptide m N°(1) | | 1.50 ppm |
| Parfum | Parfum (Fragrance) | qsp |
| Colorant | | qsp |

Les constituants de la phase A et de la phase B sont chauffés séparément à 65°C ; la phase B est incorporée à la phase A sous agitation. La température du mélange est montée à 83°C puis il est refroidi jusqu'à une température d'inversion de phase. La phase C est ensuite additionnée. L'actif est incorporé lorsque la température atteint moins de 40°C. Il est alors possible d'ajouter des parfums et/ou des colorants.

### 3. Gel Raffermissant - Amincissant - anti-cellulite

| ***Noms commerciaux*** | ***Noms INCI*** | ***% massique*** |
|---|---|---|
| Carbopol Ultrez 10 (2%) | Carbomer | 25.00 |
| Eau déminéralisée | Aqua (Water ) | Qsp |
| DUB DIOL | Methyl Propanediol | 3.00 |
| EDTA | Tetrasodium EDTA | 0.10 |
| Glydant Plus Liquid | DMDM Hydantoïn (and) Iodopropynyl butylcarbamate | 0.20 |
| Peptide ID N°(1) | | 1.25 ppm |
| TEA | Triethanolamine | 0.50 |
| Parfum | Parfum (Fragrance) | Qsp |
| Colorant hydrosoluble | | Qsp |

Le gel de Carbopol est préparé à 2 %. Les ingrédients sont ajoutés dans l'ordre énuméré ci-dessus, sous agitation. Le mélange est ensuite neutralisé avec la TEA. Le parfum et les colorants sont ajoutés si nécessaire.

## Revendications

1. Utilisation de fragments de protéines de la famille des UCP, en tant qu'agent actif, seul ou en association avec au moins un autre agent actif, dans ou pour la préparation d'une composition dermatologique et/ou cosmétique, **caractérisé en ce que** ledit agent actif de la famille des UCP est un peptide choisi parmi les peptides correspondant à la séquence ID N° :
(1) Pro Leu Asp Thr Ala Lys Val Arg Leu Gln
(2) Pro Thr Glu Val Ala Lys Val Arg Phe Gln
(3) Pro Thr Asp Val Ala Lys Val Arg Leu Gln
(4) Pro Thr Glu Val Ala Lys Val Arg Leu Gln
(5) Pro Thr Asp Val Ala Lys Val Ars Phe Gln
(6) Pro Val Asp Val Val Lys Thr Arg Phe Ile
(7) Pro Val Asp Val Val Lys Tyr Arg Tyr Met
(8) Pro Val Asp Val Val Lys Thr Arg Phe Met
(9) Pro Val Asp Val Val Lys Thr Arg Tyr Ile.

2. Utilisation selon la revendication 1 **caractérisée en ce que** le peptide correspond à la séquence ID N° (1), c'est-à-dire la séquence Pro-Leu-Asp-Thr- Ala-Lys-Val-Arg-Leu-Gln.

3. Utilisation selon l'une quelconque des revendications 1 à 2, **caractérisée en ce que** le peptide est choisi parmi les peptides dont au moins un groupement fonctionnel est protégé par un groupement protecteur, ce groupement protecteur étant soit une acylation ou une acétylation de l'extrémité amino-temainale, soit sur une amidation ou une estérification de l'extrémité carboxy-terminale, soit les deux.

4. Utilisation selon l'une quelconque des revendications précédentes **caractérisée en ce que** l'agent actif est présent, dans la composition, à une concentration comprise entre 0,05 et 500 ppm environ, et préférentiellement à une concentration comprise entre 0,1 et 50 ppm environ par rapport au poids total de la préparation finale.

5. Utilisation selon l'une quelconque des revendications précédentes **caractérisée en ce que** l'agent actif est préalablement solubilisé dans un ou plusieurs solvants cosmétiquement nu pharmaceutiquement acceptables comme l'eau, l'éthanol, le propylène glycol, le butylène glycol, le dipropylène glycol, les diglycols éthoxylés ou propoxylés, les polyols cycliques, la vaseline, une huile végétale ou tout mélange de ces solvants.

6. Utilisation selon l'une quelconque des revendications précédentes **caractérisée en ce que** l'agent actif est préalablement solubilisé dans on vecteur cosmétique ou pharmaceutique comme les liposomes ou adsorbés sur des polymères organiques poudreux, des supports minéraux comme les talcs et bentonites, et plus généralement solubilisés dans, ou fixés sur, tout vecteur cosmétiquement ou pharmaceutiquement acceptable.

7. Utilisation d'une quantité efficace d'au moins un agent actif tel que défini selon l'une quelconque des revendications 1 à 6, dans ou pour la préparation d'une composition; le composé ou la composition étant destinés au traitement de la cellulite et/ou de la peau d'orange; et/ou afin de réduire, éliminer ou prévenir les surcharges graisseuses souscutanées.

8. Composition cosmétique et/ou dermatologique et/ou pharmaceutique **caractérisée en ce qu'**elle contient, dans un milieu acceptable, comme principe actif, au moins un peptide de la famille des UCP et choisi parmi les peptides correspondant à la séquence ID N° :
(1) Pro Leu Asp Thr Ala Lys Val Arg Leu Gln
(2) Pro Thr Glu Val Ala Lys Val Arg Phe Gln
(3) Pro Thr Asp Val Ala Lys Val Arg Leu Gln
(4) Pro Thr Glu Val Ala Lys Val Arg Leu Gln
(5) Pro Thr Asp Val Ala Lys Val Arg Phe Gln
(6) Pro Val Asp Val Val Lys Thr Arg Phe Ile
(7) Pro Val Asp Val Val Lys Thr Arg Tyr Met
(8) Pro Val Asp Val Val Lys Thr Arg Phe Met
(9) Pro Val Asp Val Val Lys Thr Arg Tyr Ile.

9. Composition selon la revendication 8, **caractérisée en ce qu'**elle se présente sous la forme d'une composition cosmétique et/ou dermatologique adaptée à l'administration par voie topique cutanée comprenant un milieu cosmétiquement ou pharmaceutiquement acceptable.

10. Composition selon l'une quelconque des revendications 8 à 9, **caractérisée en ce qu'**elle se présente sous forme d'une solution aqueuse, hydroalcoolique ou huileuse ou sous la forme d'une émulsion huile-dans-eau, eau-dans-huile ou émulsions multiples ou sous forme de crèmes, de suspensions, ou encore poudres; ces compositions pouvant être plus ou moins fluides ou solides et avoir l'aspect d'une crème, d'une lotion, d'un lait, d'un sérum, d'une pommade, d'un gel, d'une pâte, d'une mousse ou d'un stick.

11. Procédé de soin cosmétique destiné à réduire, éliminer et/ou prévenir les surcharges graisseuses sous-cutanées, et/ou destiné à lutter contre la cellulite, et/ou à lutter contre le phénomène de peau d'orange, **caractérisé en ce que** l'on applique sur la surfacé de la peau une quantité efficace d'une composition telle que définie selon l'une quelconque des revendications 8 à 10.

## Claims

1. A use of fragments of proteins from the UCP family, as an active principle, alone or in combination with at least another active principle, in or for the preparation of a dermatologic and/or cosmetic composition, **characterised in that** said active principle from the UCP family is a peptide selected from the peptides corresponding to the sequence ID No.:
(1) Pro Leu Asp Thr Ala Lys Val Arg Leu Gln
(2) Pro Thr Glu Val Ala Lys Val Arg Phe Gln
(3) Pro Thr Asp Val Ala Lys Val Arg Leu Gln
(4) Pro Thr Glu Val Ala Lys Val Arg Leu Gln
(5) Pro Thr Asp Val Ala Lys Val Arg Phe Gln
(6) Pro Val Asp Val Val Lys Thr Arg Phe Ile
(7) Pro Val Asp Val Val Lys Thr Arg Tyr Met
(8) Pro Val Asp Val Val Lys Thr Arg Phe Met
(9) Pro Val Asp Val Val Lys Thr Arg Tyr Ile.

2. The use according to claim 1, **characterised in that** the peptide corresponds to the sequence ID No.(1), that is the Pro-Leu-Asp-Thr- Ala-Lys-Val-Arg-Leu-Gln sequence.

3. The use according to any of claims 1 to 2, **characterised in that** the peptide is selected from the peptides at least one functional group thereof being protected by a protecting group, this protecting group being either an acylation or an acetylation of the amino terminus, or an amidation or an esterification of the carboxy terminus, or both.

4. The use according to any of the preceding claims, **characterised in that** the active principle is present, in the composition, at a concentration between about 0.05 and 500 ppm, and preferably at a concentration between about 0.1 and 50 ppm with respect to the total weight of the final preparation.

5. The use according to any of the preceding claims, **characterised in that** the active principle is solubilised beforehand in one or more cosmetically or pharmaceutically acceptable solvents such as water, ethanol, propylene glycol, butylene glycol, dipropylene glycol, ethoxylated or propoxylated diglycols, cyclic polyols, vaseline, vegetable oil or any mixture of these solvents.

6. The use according to any of the preceding claims, **characterised in that** the active principle is solubilised beforehand in a cosmetic or pharmaceutical carrier such as liposomes or adsorbed onto powdery organic polymers, mineral supports such as talcs and bentonites, and more generally solubilised in, or affixed on, any cosmetically or pharmaceutically acceptable carrier.

7. The use of an efficient amount of at least one active principle such as defined in any of claims 1 to 6, in or for the preparation of a composition; the compound or composition being intended for treating cellulite and/or orange peel skin; and/or for reducing, removing or preventing the subcutaneous excess fat.

8. A cosmetic and/or dermatologic and/or pharmaceutical composition, **characterised in that** it contains, in an acceptable medium, as an active principle, at least one peptide from the UCP family and selected from peptides corresponding to the sequence ID N°:
(1) Pro Leu Asp Thr Ala Lys Val Arg Leu Gln
(2) Pro Thr Glu Val Ala Lys Val Arg Phe Gln
(3) Pro Thr Asp Val Ala Lys Val Arg Leu Gln
(4) Pro Thr Glu Val Ala Lys Val Arg Leu Gln
(5) Pro Thr Asp Val Ala Lys Val Arg Phe Gln
(6) Pro Val Asp Val Val Lys Thr Arg Phe Ile
(7) Pro Val Asp Val Val Lys Thr Arg Tyr Met
(8) Pro Val Asp Val Val Lys Thr Arg Phe Met
(9) Pro Val Asp Val Val Lys Thr Arg Tyr Ile.

9. The composition according to claim 8, **characterised in that** it is as a cosmetic and/or dermatologic composition suitable for cutaneous topical administration including a cosmetically or pharmaceutically acceptable medium.

10. The composition according to any of claims 8 to 9, **characterised in that** it is as an aqueous, hydroalcoholic or oily solution or as an oil in water, water in oil emulsion, or multiple emulsions or as creams, suspensions, or even powders; wherein these compositions can be more or less fluid or solid and take on the appearance of a cream, lotion, milk, serum, ointment, gel, paste, foam or stick.

11. A cosmetic care method for reducing, removing and/or preventing the subcutaneous excess fat, and/or controlling cellulite, and/or controlling the orange peel skin phenomenon, **characterised in that** an efficient amount of the composition such as defined in any of claims 8 to 10 is applied onto the surface of the skin.

## Patentansprüche

1. Verwendung von Proteinfragmenten der UCP-Familie als alleiniger Wirkstoff oder in Kombination mit mindestens einem anderen Wirkstoff in der oder für die Zubereitung einer dermatologischen und/oder kosmetischen Zusammensetzung, **dadurch gekennzeichnet, dass** der Wirkstoff aus der Familie der UCP ein Peptid ist, das aus den Peptiden ausgewählt ist, die der Sequenz ID Nr.:
(1) Pro Leu Asp Thr Ala Lys Val Arg Leu Gln
(2) Pro Thr Glu Val Ala Lys Val Arg Phe Gln
(3) Pro Thr Asp Val Ala Lys Val Arg Leu Gln
(4) Pro Thr Glu Val Ala Lys Val Arg Leu Gln
(5) Pro Thr Asp Val Ala Lys Val Arg Phe Gln
(6) Pro Val Asp Val Val Lys Thr Arg Phe Ile
(7) Pro Val Asp Val Val Lys Thr Arg Tyr Met
(8) Pro Val Asp Val Val Lys Thr Arg Phe Met
(9) Pro Val Asp Val Val Lys Thr Arg Tyr Ile entsprechen.

2. Verwendung nach Anspruch 1, **dadurch gekennzeichnet, dass** das Peptid der Sequenz ID Nr. (1) entspricht, das heißt, der Sequenz Pro-Leu-Asp-Thr-Ala-Lys-Val-Arg-Leu-Gln.

3. Verwendung nach einem der Ansprüche 1 bis 2, **dadurch gekennzeichnet, dass** das Peptid aus den Peptiden ausgewählt ist, bei denen mindestens eine funktionelle Gruppe von einer Schutzgruppe geschützt wird, wobei diese Schutzgruppe entweder eine Acylierung oder eine Acetylierung des amino-terminalen Endes oder eine Amidierung oder eine Veresterung des carboxyterminalen Endes oder beides ist.

4. Verwendung nach einem der vorangehenden Ansprüche, **dadurch gekennzeichnet, dass** der Wirkstoff in der Zusammensetzung in einer Konzentration zwischen zirka 0,05 und 500 ppm inklusive und vorzugsweise in einer Konzentration zwischen zirka 0,1 und 50 ppm inklusive im Verhältnis zum Gesamtgewicht der endgültigen Zubereitung vorhanden ist.

5. Verwendung nach einem der vorangehenden Ansprüche, **dadurch gekennzeichnet, dass** der Wirkstoff zuvor in einem oder mehreren kosmetisch oder pharmazeutisch akzeptablen Lösungsmitteln wie Wasser, Ethanol, Propylenglycol, Butylenglycol, Dipropylenglycol, ethoxylierte oder propoxylierte Diglycole, zyklische Polyole, Vaseline, einem Pflanzenöl oder jedem Gemisch dieser Lösungsmittel solubilisiert werden.

6. Verwendung nach einem der vorangehenden Ansprüche, **dadurch gekennzeichnet, dass** der Wirkstoff zuvor in einem kosmetischen oder pharmazeutischen Vektor wie die Libosome solubilisiert oder auf pulvrigen organischen Polymeren, mineralischen Trägern wie die Talke und Bentonite adsorbiert wird, und allgemein solubilisiert in oder fixiert auf jedem kosmetisch oder pharmazeutisch akzeptablen Vektor.

7. Verwendung einer wirksamen Menge mindestens eines Wirkstoffs, wie nach einem der Ansprüche 1 bis 6 definiert, in einer oder für eine Zubereitung einer Zusammensetzung, wobei die Verbindung oder die Zusammensetzung zur Behandlung der Cellulite und/oder der Orangenhaut bestimmt ist und/oder zur Reduzierung, Beseitigung oder Vorbeugung subkutaner Fettüberladungen.

8. Kosmetische und/oder dermatologische und/oder pharmazeutische Zusammensetzung **dadurch gekennzeichnet, dass** sie in einem akzeptablen Medium als Wirkstoff mindestens ein Peptid aus der Familie der UCP und ausgewählt aus den Peptiden enthält, die der Sequenz ID Nr.:
(1) Pro Leu Asp Thr Ala Lys Val Arg Leu Gln
(2) Pro Thr Glu Val Ala Lys Val Arg Phe Gln
(3) Pro Thr Asp Val Ala Lys Val Arg Leu Gln
(4) Pro Thr Glu Val Ala Lys Val Arg Leu Gln
(5) Pro Thr Asp Val Ala Lys Val Arg Phe Gln
(6) Pro Val Asp Val Val Lys Thr Arg Phe Ile
(7) Pro Val Asp Val Val Lys Thr Arg Tyr Met
(8) Pro Val Asp Val Val Lys Thr Arg Phe Met
(9) Pro Val Asp Val Val Lys Thr Arg Tyr Ile entsprechen.

9. Zusammensetzung nach Anspruch 8, **dadurch gekennzeichnet, dass** sie sich in Form einer kosmetischen und/oder dermatologischen Zusammensetzung präsentiert, die für die Verabreichung auf kutanem topischem Weg geeignet ist und ein kosmetisch oder pharmazeutisch akzeptables Medium umfasst.

10. Zusammensetzung nach einem der Ansprüche 8 bis 9, **dadurch gekennzeichnet, dass** sie sich in Form einer wässrigen, hydroalkoholischen oder öligen Lösung oder in der Form einer Öl-in-Wasser-, Wasser-in-Öl-Emulsion oder multipler Emulsionen oder in Form von Cremes, Suspensionen oder auch Pulvern präsentiert, wobei diese Zusammensetzungen mehr oder weniger flüssig oder fest sein können und wie eine Creme, eine Lotion, eine Milch, ein Serum, eine Pomade, ein Gel, eine Paste, ein Schaum oder ein Stick aussehen können.

11. Kosmetisches Pflegeverfahren, das dazu bestimmt ist, subkutane Fettüberlagerungen zu reduzieren, zu beseitigen und/oder ihnen vorzubeugen und/oder dazu bestimmt ist, Cellulite zu bekämpfen und/oder die Erscheinung der Orangenhaut zu bekämpfen, **dadurch gekennzeichnet, dass** eine wirksame Menge einer Zusammensetzung wie nach einem der Ansprüche 8 bis 10 definiert auf die Oberfläche der Haut aufgetragen wird.
